**Europäisches Patentamt**

(19) **European Patent Office**

**Office européen des brevets**

(11) Publication number : **0 313 617 B1**

# (12) EUROPEAN PATENT SPECIFICATION

(45) Date of publication of patent specification :
**12.08.92 Bulletin 92/33**

(51) Int. Cl.$^5$ : **A61K 31/22, A61K 9/10**

(21) Application number : **88904135.6**

(22) Date of filing : **15.04.88**

(86) International application number :
**PCT/US88/01278**

(87) International publication number :
**WO 88/08301 03.11.88 Gazette 88/24**

(54) **LIPID EMULSION AND METHOD FOR INTRAVENOUS INFUSION.**

(30) Priority : **22.04.87 US 41165**

(43) Date of publication of application :
**03.05.89 Bulletin 89/18**

(45) Publication of the grant of the patent :
**12.08.92 Bulletin 92/33**

(84) Designated Contracting States :
**BE CH DE FR GB IT LI SE**

(56) References cited :
**US-A- 3 169 094**
**US-A- 4 280 996**
**US-A- 4 678 808**

(73) Proprietor : **BAXTER INTERNATIONAL INC. (a Delaware corporation)**
**One Baxter Parkway**
**Deerfield Illinois 60015 (US)**

(72) Inventor : **COTTER, Richard**
**1717 Cedar Glen Drive**
**Libertyville, Il 60048 (US)**

(74) Representative : **Bassett, Richard Simon et al**
**ERIC POTTER & CLARKSON St. Mary's Court**
**St. Mary's Gate**
**Nottingham NG1 1LE (GB)**

## Description

The present invention relates generally to lipid emulsion systems. Specifically, the present invention relates to lipid emulsion systems for intravenous infusion.

It is known to intravenously infuse lipid emulsions into patients. Typically, lipid emulsions comprise: an aqueous environment; an emulsifier; and a glyceride oil component. Present commercial lipid emulsions, such as, for example, TRAVAMULSION[R], INTRALIPID[R], and LYPOSYN[R], utilize an excess amount of phospholipids as an emulsifier in their formulations. For example, these formulations typically have a phospholipid to glyceride oil ratio, by weight, of approximately 0.12 to 0.06. The excess phospholipids result in a fraction of the expected emulsion vesicles as well as a single and multilemelar liposome fraction.

As stated above, these lipid emulsions are designed to be infused into a patient. Because of the use of excess phospholipids, removal of the lipid emulsions from the blood stream may not proceed at a sufficiently fast rate. It is believed that removal of the emulsion particles from the blood stream is by a pathway having a half life (T 1/2) of up to approximately 1 hour. However, removal of the liposome particles from the blood stream is by another pathway, having an extremely long T 1/2 of 2 days. (See Untracht, S.H., Intravascular Metabolism of an Artificial Transporter of Triacylglycerols, Biochim Biophys Acta 711(1): 176-92, 1982.) Because the liposome particles are removed from the blood stream via this second pathway that has a long T 1/2 hyperlipidemia is observed in many patients after parenteral lipid emulsion infusions. The inventor of the present invention believes that it is the liposomes, that use the longer T 1/2 pathway, that produce the hyperlipidemia observed in patients who receive lipid emulsion infusions.

Hyperlipidemia is a condition wherein the patient has too much lipid in his blood. This results in an increase in the risk of cardio-vascular disease gastrointestinal disturbances, heptosplenomegaly, impared hepatic function, anemia, thromobocytopenia, prolonged clotting time, spontaneous bleeding, and respiratory complications. This is especially true in neonates and infants, wherein hyperlipidemia is a dangerous condition that can cause,respiratory failure. Indeed, many doctors will not prescribe lipid emulsions for neonates and infants due to the fear of hyperlipidemia and accompanying respiratory failure.

It has been found that lipid emulsion made using a reduced phospholipid to oil weight ratio at the critical concentration, i.e., the concentration where the amount necessary for complete emulsification of the glyceride oil with the emulsifier takes place, greatly reduces the liposomal content and thus avoids hyperlipidemia in patients.

US-A-3 169 094 discloses intravenous emulsions in which, because egg phosphatides are used as the emulsifier and the lipid is soy bean oil, improved results were apparently obtained. There was no disclosure concerning the importance of regulating the ratio of the emulsifiers to the oil. US-A-4 280 996 discloses intravenous lipid emulsions in which the ratio of phospholipid emulsifiers to the oil could be from 0.04 to 0.25 but there was no disclosure of the relevance of the ratio to the clinical performance of the compositions and ratios below 0.04 were neither disclosed or suggested.

The present invention provides a lipid emulsion for nutritional support of a patient, as well as a lipid emulsion system for delivering therapeutic agents whilst avoiding hyper lipidaemia. The lipid emulsion comprises water, an emulsifier, and a glyceride oil component. The weight ratio of the emulsifier to glyceride oil is 0.04 to 0.01. It has been found that intravenous lipid emulsion having a weight ratio of emulsifier to glyceride oil of 0.04 to 0.01 are more rapidly metabolically utilized.

Preferably, the emulsifier is a phospholipid. The glyceride oil can be mono-, di-, or tri-. Preferably, the lipid emulsion includes an osmotic agent. Preferably, the osmotic agent can include, for example, glycerol, glucose, xylitol, or sorbitol. Preferably, a sufficient amount of an osmotic agent is used to produce a solution with a final osmolarity of 280 to 300 milliosmoles.

Accordingly, an advantage of the present invention is to provide an improved lipid emulsion system.

Another advantage of the present invention is that it provides a more rapidly metabolized lipid nutritional supply.

A further advantage of the present invention is that it provides a lipid emulsion system for delivering lipid soluble drugs.

A still further advantage of the present invention is that it provides a lipid emulsion that reduces the risk of hyperlipidemia in patients who receive the lipid emulsion.

Additional features and advantages of the present invention are described in, and will be apparent from, the detailed description of the presently preferred embodiments and from the drawings.

Figure 1 illustrates the emulsion kinetics at different phospholipid to oil ratios through a plot of the plasma triglyceride concentration versus time.

Figure 2 illustrates a plot of the plasma phospholipid concentration versus time at different phospholipid to oil ratios.

Figure 3 illustrates a plot of the plasma triglyceride level versus time.

The present invention provides a lipid emulsion for nutritional support, as well as, a lipid emulsion system for delivering a therapeutic agent. The lipid emulsion includes an emulsifier, a glyceride oil component, an aqueous environment, and preferably an osmotic agent. Preferably, the emulsifier is a phospholipid. Preferably, the phospholipid is chosen among egg phosphatides, soybean phosphatides, or phosphatides of marine origin. Although the oil can be a mono-, di-, or tri-, glyceride, preferably, the oil is triglyceride. Preferably the triglyceride oil is chosen among soybean, safflower, marine, black currant seed, borage oil, palm kernel oil, or coconut oil. Preferably the lipid emulsion contains 10 to 50% by weight oil. Preferably, the osmotic agent is chosen among glycerol, glucose, sorbitol, or xylitol. Preferably, sufficient osmotic agent is used so that a solution with a final osmolarity of 280 to 300 milliosmoles is achieved.

The emulsifier to triglyceride oil weight ratio of the lipid emulsion of the present invention is less than 0.04. Preferably the emulsifier to triglyceride weight ratio is 0.04 to 0.01. As set forth in detail below, by utilizing a low emulsifier to triglyceride oil ratio a greatly reduced liposomal content is observed with a reduction of risk of hyperlipidemia in patients. Due to the reduction in liposomal content, a more rapidly metabolized lipid emulsion is achieved.

The lipid emulsions are recommended clinically for nutritional support to be used at dosages up to 2.5 g/kg/24 hours for adults and up to 4 g/kg/24 hours for children. However, the dosage. levels of these emulsions are recommendations and each patient must be monitored for the build up of emulsions and free fatty acids during infusion to assure the safety of such therapies.

Examples of the present invention will now be set forth to illustrate the advantages of using a lipid emulsion having a low phospholipid to oil weight ratio, i.e. a ratio equal to or below 0.04. The metabolism of three types of 10% intravenous lipid emulsion was evaluated using TRAVAMULSION® available from Travenol Laboratories, Inc., Deerfield, Illinois. The emulsions were prepared with different phospholipid to triglyceride oil ratios and then infused into beagle dogs. In addition to infusing only lipids, the three emulsions were mixed with amino acids and dextrose to determine their stability in a typical total parenteral nutrition (TPN) formulation. The TPN solutions were also infused into beagles.

The 10% intravenous lipid emulsions evaluated were: 10% TRAVAMUSLION[R] with 1.2% egg phosphatide (0.12 phospholipid to oil ratio) (comparative example); 10% TRAVAMULSION[R] with 0.7% egg phosphatide (0.07 phospholipid to oil ratio) (comparative example); and 10% TRAVAMULSION[R] with 0.4% egg phosphatide (0.04 phospholipid to oil ratio). As stated above, to determine their stability in a TPN regime, the lipid emulsions were also mixed with dextrose and amino acids. The dextrose was a 10% dextrose solution and the amino acids solution was 8.5% TRAVASOL® with electrolytes available from Travenol Laboratories, Inc., Deerfield, Illinois.

Three male beagle dogs weighing approximately 7 to 13 kg were used in the study. The aninals were obtained and housed in accordance with Good Laboratory Practices.

Phase 1 of the study was conducted over a three week period wherein the three male beagle dogs were infused with a lipid emulsion, for a four-hour period, for three consecutive days each week. All the dogs were infused with a 10% intravenous lipid emulsion at a lipid dose of 2 g/kg. During week one: beagle 102-4 received a lipid emulsion containing a 0.04 phospholipid to oil weight ratio; beagle 110-4 received a lipid emulsion containing a 0.07 phospholipid to oil weight ratio; and beagle 111-4 received a lipid emulsion containing a 0.12 phospholipid to oil weight ratio. During week 2: beagle 110-4 received a lipid emulsion having a 0.04 phospholipid to oil ratio; beagle 111-4 received a lipid emulsion having a 0.07 phospholipid to oil ratio; and beagle 102-4 received a lipid emulsion having a 0.12 phospholipid to oil ratio. During week three: beagle 111-4 received a lipid emulsion having a 0.04 phospholipid to oil ratio; beagle 102-4 received a lipid emulsion having a 0.07 phospholipid to oil ratio; and beagle 110-4 received a lipid emulsion having a 0.12 phospholipid to oil ratio. Accordingly, at the end of the three week study, each dog received each of the three lipid emulsions. (Phase 1 is summarized in Table I below.)

Table I

PHASE 1

10% TRAVAMULSION$^R$

| | 0.04 Phospholipid:Oil | | | 0.07 Phospholipid:Oil | | |
|---|---|---|---|---|---|---|
| | Day 1 | Day 2 | Day 3 | Day 1 | Day 2 | Day 3 |
| Week 1 | Dog 102-4 | Dog 102-4 | Dog 102-4 | Dog 110-4 | Dog 110-4 | Dog 110-4 |
| Week 2 | Dog 110-4 | Dog 110-4 | Dog 110-4 | Dog 111-4 | Dog 111-4 | Dog 111-4 |
| Week 3 | Dog 111-4 | Dog 111-4 | Dog 111-4 | Dog 102-4 | Dog 102-4 | Dog 102-4 |

Table I Cont'd

PHASE 1

10% TRAVAMULSION$^R$

| | 0.12 Phospholipid:Oil | | |
|---|---|---|---|
| | Day 1 | Day 2 | Day 3 |
| Week 1 | Dog 111-4 | Dog 111-4 | Dog 111-4 |
| Week 2 | Dog 102-4 | Dog 102-4 | Dog 102-4 |
| Week 3 | Dog 110-4 | Dog 110-4 | Dog 110-4 |

Blood samples (approximately 5 ml) were taken from the dogs during and after the lipid emulsion infusions at 0, 0.5, 1, 2, 4, 4, 4.5, 5, 6, 7, 8, and 72 hours after the start of lipid emulsion infusions. The serum was analyzed for triglyceride content by nephelometry and an aliquot of serum was submitted to the Clinical Laboratory Services of Travenol Laboratories for measurement of the phospholipid content.

Phase 2 of the evaluation involved an additional week of testing. The same three beagle dogs were infused, for a four-hour period, for three consecutive days with a three in one solution of a lipid, protein, and carbohydrate at a dose of 2 g/kg each. During this additional week, beagle 102-4 received a three in one solution having 0.04 egg phospholipid to oil weight ratio; beagle 110-4 received a three in one solution having 0.07 egg phospholipid to oil weight ratio; and beagle 111-4 received a three in one solution having a 0.12 egg phospholipid to oil weight ratio. (Phase 2 is summarized in Table II below.) As in Phase 1, blood samples were taken from the dogs and analyzed for triglyceride and phospholipid content.

Table II

PHASE 1

10% TRAVAMULSION ®

| Solution | Day 1 | Day 2 | Day 3 |
|---|---|---|---|
| 0.04 | | | |
| Phospholipid:Oil | Dog 102-4 | Dog 102-4 | Dog 102-4 |
| 0.07 | | | |
| Phospholipid:Oil | Dog 110-4 | Dog 110-4 | Dog 110-4 |
| 0.12 | | | |
| Phospholipid:Oil | Dog 111-4 | Dog 111-4 | Dog 111-4 |

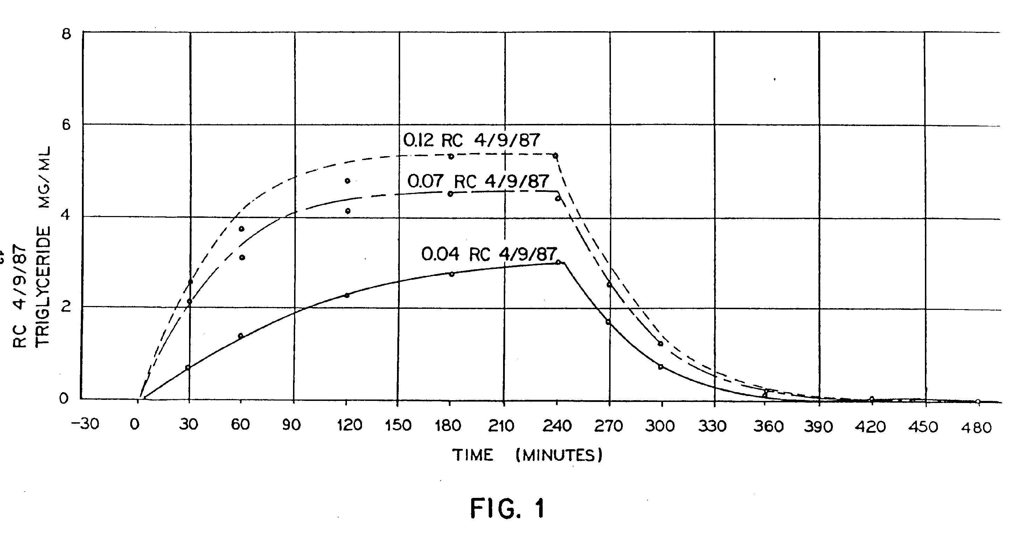

The values for the triglyceride concentration in the blood of the beagles were analyzed using a mathematical model designed to evaluate the kinetics of emulsion metabolism. A plot of triglyceride concentration vs time is illustrated in Figure 1. In Figure 1 the plot points were determined by plotting the observed valves for all the readings and determining the mean - the lipid emulsion with a 0.04 phospholipid to oil weight ratio is represented by a solid line; the lipid emulsion with a 0.07 phospholipid to oil weight ratio is represented by a sectional line; and the lipid emulsion with 0.12 phospholipid to oil weight ratio is represented by a dotted line. The analysis is summarized below. A number of kinetic parameters indicative of metabolism were evaluated and are indicated. Table III sets forth the geometric means for the kinetic parameters during the infusion period.

Table III

Statistical Comparison of Kinetic Parameters

| | Kinetic Parameter | Phospholipid to Oil Ratios | | |
|---|---|---|---|---|
| | | 0.04 | 0.07 | 0.12 |
| | BL | 0.000 | 0.000 | 0.002 |
| Infusion | SS | 3.12 | 4.54 | 5.36 |
| Phase | MPR | 0.024 | 0.073 | 0.089 |
| | THM | 70.7 | 33.9 | 32.8 |
| Elimination | EIC | 3.20 | 4.49 | 5.03 |
| | MMP | 0.94 | 1.31 | 0.96 |
| Phase | MER | 0.055 | 0.070 | 0.077 |
| | HL | 20.1 | 22.0 | 22.6 |
| | THI | 31.9 | 34.9 | 35.8 |
| | TBL | 254 | 290 | 270 |

## Table III Cont'd
### Statistical Comparison of Kinetic Parameters

|  | Kinetic Parameter | Statistical Comparison ($a = 0.05$) |
|---|---|---|
|  | BL | NS |
| Infusion | SS | NS |
| Phase | MPR | L $>$ M, H |
|  | THM | NS |
| Elimination | EIC | NS |
| Phase | MMP | NS |
|  | MER | NS |
|  | HL | NS |
|  | THI | NS |

BL = base line value of tri-glyceride before infusion.

NS=Not Significant

L =Low  P:O Ratio

```
SS   =  steady state levels of      M =Medium  P:O Ratio
        triglyceride.               H = High   P:O Ratio
MPR  =  maximum pick up rate -
        initial rate of increase
        of triglyceride at early
        time points.
THM  =  time to reach 1/2 steady state.
EIC  =  elimination phase initial
        concentration.
MMP  =  Michaelis-Menten parameter
        concentration at which the
        elimination rate if half
        maximum.
MER  =  maximum elimination rate -
        elimination rate at beginning
        of the elimination period.
HL   =  half life in the first order
        region of the elimination period.
THI  =  time to reach half of the initial
        concentration.
TBL  =  time to reach the baseline value.
```

Statistical analysis reveals that the maximum pick-up rate, i.e. the initial rate of increase, in triglyceride level was significantly lower for the 0.04 phospholipid to oil weight ratio lipid emulsion group relative to the 0.07 and 0.12 phospholipid to oil weight ratio lipid emulsion groups. This demonstrates that the initial elimination rate was fastest for the 0.04 ratio group. The data further illustrates a trend which indicates that steady state triglyceride levels are proportional to phospholipid to oil ratios and that the THM (time to reach 1/2 steady state) is greater for the 0.04 ratio group, which is consistent with the slower rise in triglycerides.

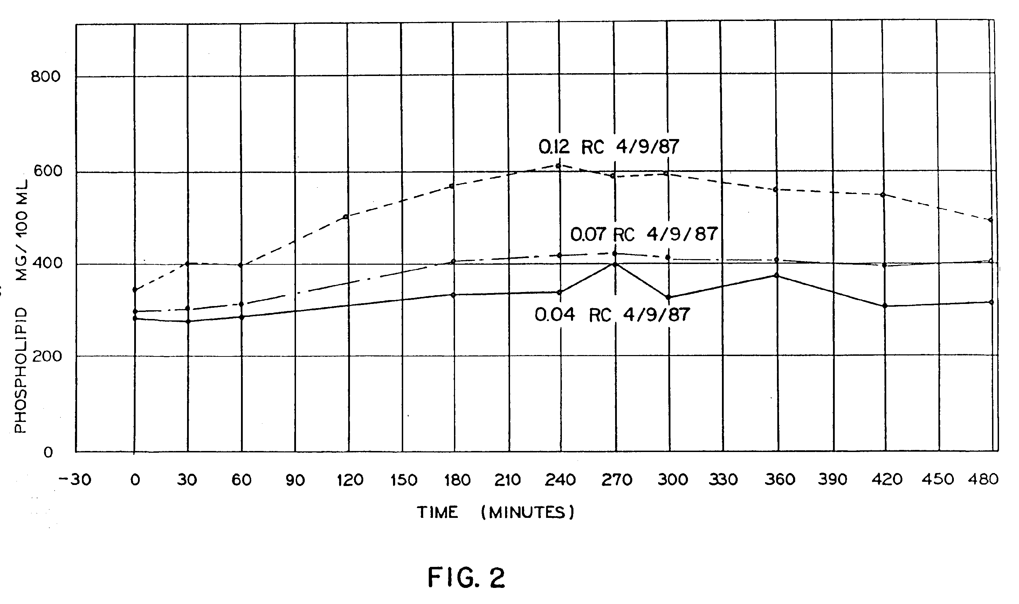

An analysis of phospholipid concentration across time reveals that there was no significant difference between the 0.04 and 0.07 ratio groups. But, from 60 minutes to 8 hours the 0.12 ratio group was significantly higher than the 0.04 and 0.07 ratio groups. (See Figure 2 that illustrates the phospholipid levels; again, 0.04 is represented by a solid line, 0.07 by a sectional line, and 0.12 by a dotted line.) Moreover, the data indicates that successive lipid emulsion infusions of lipid emulsions having 0.04 and 0.07 phospholipid to oil weight ratio will not cause a rise in the baseline levels of plasma phospholipids which was observed with the 0.12 phospholipid to oil ratio group.

The lipid emulsion particle size (particle volume-surface mean diameter) was also analyzed and is set forth in Table IV.

## Table IV

| Sample Identification | Volume Surface Mean Diameters DVS(nm) |
|---|---|
| Emulsion with 0.12 phospholipid to oil ratio* | 204.5 |
| Emulsion with 0.07 phospholipid to oil ratio | 250.8 |
| Emulsion with 0.04 phospholipid to oil ratio | 268.6 |
| Mixture with 0.12 phospholipid to oil ratio | 197.7 |
| Mixture with 0.12 phospholipid to oil ratio | 204.7 |
| Mixture with 0.12 phospholipid to oil ratio | 196.2 |
| Mixture with 0.07 phospholipid to oil ratio | 241.3 |
| Mixture with 0.07 phospholipid to oil ratio | 240.3 |
| Mixture with 0.07 phospholipid to oil ratio | 249.2 |
| Mixture with 0.04 phospholipid to oil ratio | 281.0 |
| Mixture with 0.04 phospholipid to oil ratio | 278.1 |
| Mixture with 0.04 phospholipid to oil ratio | 270.4 |

*by weight

Particle size increased with a decreasing phospholipid to oil ratio. A large particle size is advantageous because it demonstrates maximum emulsification.

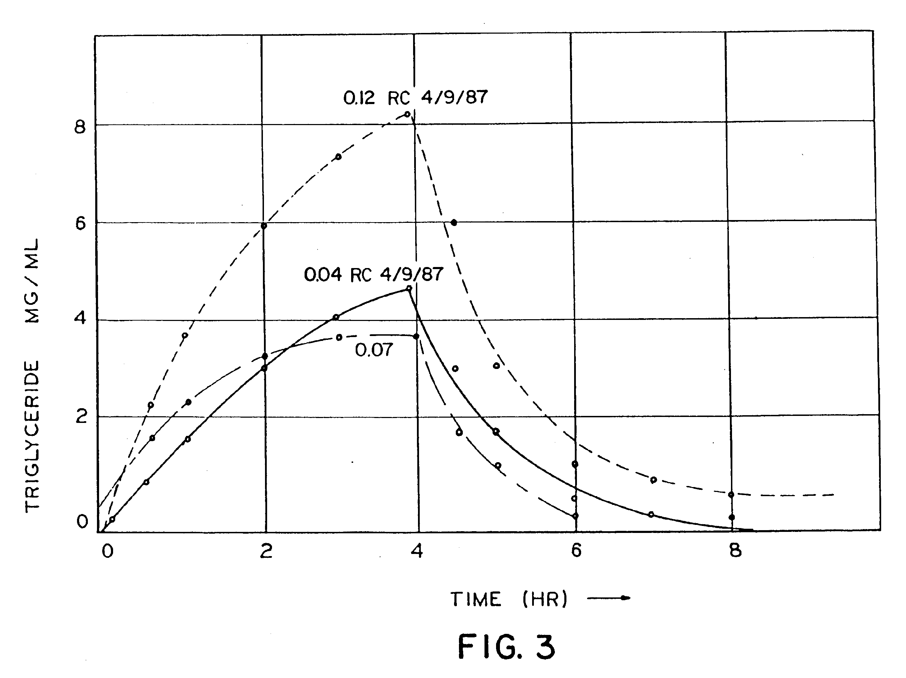

Each of the lipid emulsions was mixed with an amino acid solution, TRAVASOL[R], and dextrose and placed in plastic infusion bags. The solution was stored at room temperature for approximately 16 hours and then infused into beagle dogs over four hours. Five ml samples were then removed and stored at 4°C until particle size was analyzed approximately 6 weeks later. Visual examination indicated no breakdown of the emulsion and particle size analysis indicated no change had occurred. Tests indicate that plasma triglyceride levels were equivalent in dogs receiving this TPN solution over four hours when compared to animals receiving only lipids. The study established that metabolism of the lipid emulsion is enhanced by lowering the phospholipid to oil ratios and that moreover, the lipid emulsions having a low phospholipid to oil ratios are stable when mixed with a TPN solution, such as amino acids and dextrose. Figure 3 illustrates a plot of the average concentrations for each lipid emulsion in the three in one infusion. Again, the 0.04 ratio solution is indicated by a solid line, 0.07 by a sectional line, and 0.12 by a dotted line.

Not only does the present invention provide an improved lipid emulsion for nutritional support, but, it also provides an improved lipid emulsion system for infusion of therapeutic agents. To this end, the present invention provides a lipid emulsion system capable of being utilized to infuse lipid soluble drugs. To overcome the unpleasant side effects associated with the intravenous injection of some drugs that are poorly soluble in water, it may be desirable to mix these drugs in a lipid emulsion. For example, Valium is typically mixed with co-solvents, such as alcohol and propylene glycol, that makes the intravenous infusion of Valium painful. However, Valium is a lipid soluble drug, and therefore, can be mixed in the lipid emulsion of the present invention and thereby, infused in a patient. Other agents that can be encapsulated in a lipid emulsion include, for example, antibiotics such as cephalosporin and antineoplastic drugs such as adriamycin.

## Claims

1. A lipid emulsion for intravenous administration whilst avoiding hyper lipidaemia comprising an emulsifier, a glyceride oil and water, characterised in that the weight ratio of emulsifier to glyceride oil is less than 0.04, and in that a composition consisting of soy bean oil, egg phosphatides, and glycerol and water having a said weight ratio of 0.01 is disclaimed.

2. The lipid emulsion of Claim 1 including an osmotic agent.

3. The lipid emulsion of Claim 1 or 2 wherein the emulsifier is a phospholipid.

4. A lipid emulsion system comprising a phospholipid emulsifier, a lipid oil, an osmotic agent and water, characterised in that the weight ratio of the phospholipid emulsifier to lipid oil is less than 0.04 and more than 0.01.

5. The lipid emulsion of Claim 2 or 4 wherein the osmotic agent is selected from glycerol, glucose, xylitol and sorbitol.

6. The lipid emulsion of Claim 2, 4 or 5 wherein sufficient osmotic agent is present to produce an osmolarity of 280 to 300 milliosmoles.

7. The lipid emulsion of any one of the preceding claims wherein the oil is selected from monoglyceride, diglyceride and triglyceride oils.

8. The lipid emulsion of any one of the preceding claim wherein the oil is or is derived from a oil selected from soybean, safflower, marine, blackcurrant seed, borage, palm kernel and coconut oils.

9. The lipid emulsion of any one of the preceding claims wherein the emulsifier is selected from egg phosphatides, phosphatides of marine origin and soybean phosphatides.

10. The lipid emulsion of any one of the preceding claims wherein the oil constitutes, by weight, 10 to 50 per cent of the emulsion.

11. The lipid emulsion system of any one of the preceding claims including a lipid soluble agent.

12. A process for preparing a composition for intravenous infusion comprising the step of adding a lipid soluble agent to a lipid emulsion according to any one of Claims 1 to 10.

13. A process for preparing a composition for intravenous infusion comprising the step of adding an osmotic agent to a lipid emulsion according to Claim 1 to produce an osmolarity of 280 to 300 milliosmoles.

14. A process for preparing a composition for intravenous infusion comprising the step of mixing a lipid emulsion according to any one of Claims 1 to 11 with an amino acid solution and a dextrose solution.

15. The use of a lipid emulsion comprising an emulsifier, a glyceride oil and water, the weight ratio of emulsifier to glyceride oil being less than or equal to 0.04, in the manufacture of a medicament for the intravenous infusion of lipids into a patient whilst avoiding hyperlipidaemia.

16. A use according to Claim 15 wherein the patient is a neonate or an infant.

## Patentansprüche

1. Lipidemulsion zur intravenösen Verabreichung unter Vermeidung von Hyperlipidämie, die einen Emulgator, ein Glyceridöl und Wasser aufweist, dadurch gekennzeichnet, daß das Gewichtsverhältnis von Emulgator zu Glyceridöl kleiner als 0,04 ist, und daß eine Zusammensetzung, die aus Sojabohnemöl, Ei-Phosphatiden und Glycerin und Wasser besteht und ein genanntes Gewichtsverhältnis von 0,01 hat, ausdrücklich ausgenommen ist.

2. Lipidemulsion nach Anspruch, die ein osmotisches Mittel enthält.

3. Lipidemulsion nach Anspruch 1 oder 2, wobei der Emulgator ein Phospholipid ist.

4. Lipidemulsionssystem das einen Phospholipid-Emulgator, ein Lipidöl, ein osmotisches Mittel und Wasser aufweist, dadurch gekennzeichnet, daß das Gewichtsverhältnis des Phospholipid-Emulgators zu Lipidöl kleiner als 0,04 und größer als 0,01 ist.

5. Lipidemulsion nach Anspruch 2 oder 4, wobei das osmotische Mittel aus Glycerin, Glucose, Xylit und Sorbit ausgewählt ist.

6. Lipidemulsion nach Anspruch 2, 4 oder 5, wobei ausreichend osmotisches Mittel vorhanden ist, um eine Osmolarität von 280-300 Milliosmolen zu erzeugen.

7. Lipidemulsion nach einem der vorhergehenden Ansprüche, wobei das Öl aus Monoglycerid-, Diglycerid- und Triglyceridölen ausgewählt ist.

8. Lipidemulsion nach einem der vorhergehenden Ansprüche, wobei das Öl ein Öl ist oder von einem Öl abgeleitet ist, das aus Sojabohnen-, Saflor-, Seetier-, Schwarze-Johannisbeersamen-, Boretsch-, Palmkern- und Kokosnußölen ausgewählt ist.

9. Lipidemulsion nach einem de vorhergehenden Ansprüche, wobei de Emulgator aus Ei-Phosphatiden, Phosphatiden, die von Seetieren stammen, und Sojabohnen-Phosphatiden ausgewählt ist.

10. Lipidemulsion nach einem der vorhergehenden Ansprüche, wobei das Öl 10-50 Gew.-% der Emulsion bildet.

11. Lipidemulsionssystem nach einem der vorhergehenden Ansprüche, das ein lipidlösliches Mittel enthält.

12. Verfahren zur Herstellung einer Zusammensetzung zur intravenösen Infusion, das den Schritt der Zugabe eines lipidlöslichen Mittels zu einer Lipidemulsion nach einem der Ansprüche 1-10 aufweist.

13. Verfahren zur Herstellung einer Zusammensetzung zur intravenösen Infusion, das den Schritt der Zugabe eines osmotischen Mittels zu einer Lipidemulsion nach Anspruch 1 aufweist, um eine Osmolarität von 280-300 Milliosmolen zu erzeugen.

14. Verfahren zur Herstellung einer Zusammensetzung zur intravenösen Infusion, das den Schritt des Vermischens einer Lipidemulsion nach einem der Ansprüche 1-11 mit einer Aminosäurelösung und einer Dextroselösung aufweist.

15. Verwendung einer Lipidemulsion, die einem Emulgator, ein Glyceridöl und Wasser aufweist, wobei das Gewichtsverhältnis von Emulgator zu Glyceridöl kleiner als oder gleich 0,04 ist, bei der Herstellung eines Medikaments zur intravenösen Infusion von Lipiden in einen Patienten unter Vermeidung von Hyperlipidämie.

16. Verwendung nach Anspruch 15, wobei der Patient ein Neugeborenes oder ein Kind ist.


## Revendications

1. Emulsion de lipides pour injection intraveineuse qui évite l'hyperlipidémie et qui comprend un émulsifiant, une huile de glycéride et de l'eau, caractérisée en ce que le rapport pondéral émulsifiant-huile de glycéride est inférieur à 0,04 et en ce qu'une composition constituée d'huile de soya, de phosphatide de l'oeuf, d'eau et de glycérol ayant un dit rapport pondéral égal à 0,01 est non revendiquée.

2. Emulsion de lipides selon la revendication 1, comprenant un agent osmotique.

3. Emulsion de lipides selon la revendication 1 ou 2, dans laquelle l'émulsifiant est un phospholipide.

4. Système d'émulsion de lipides comprenant un phospholipide émulsifiant, une huile de lipides, un agent osmotique et de l'eau, caractérisé en ce que le rapport pondéral du phospholipide émulsifiant à l'huile de lipide est inférieur à 0,04 et supérieur à 0,01.

5. Emulsion de lipides selon la revendication 2 ou 4, dans laquelle l'agent osmotique est choisi parmi le glycérol, le glucose, le xylitol et le sorbitol.

6. Emulsion de lipides selon la revendication 2, 4 ou 5, dans laquelle il existe suffisamment d'agent osmotique pour produire une osmolarité comprise entre 280 et 300 milliosmoles.

7. Emulsion de lipides selon l'une quelconque des revendications précédentes, dans laquelle l'huile est choisie parmi les huiles de monoglycérides, de glycérides et de triglycérides.

8. Emulsion de lipides selon l'une quelconque des revendications précédentes, dans laquelle l'huile est une huile choisie parmi l'huile de soya, l'huile de carthame, l'huile marine, l'huile de cassis, l'huile de bourrache, l'huile de palmiste et l'huile de noix de coco ou est un dérivé d'une de ces huiles.

9. Emulsion de lipides selon l'une quelconque des revendications précédentes, dans laquelle l'émulsifiant est choisi parmi les phosphatides de l'oeuf, les phosphatides d'origine marine et les phosphatides de soya.

10. Emulsion de lipides selon l'une quelconque des revendications précédentes, dans laquelle l'huile constitue de 10 à 50% en poids de l'émulsion.

11. Système d'émulsion de lipides selon l'une quelconque des revendications précédentes, comprenant un agent soluble dans les lipides.

12. Procédé de préparation d'une composition pour injection intraveineuse comprenant l'étape d'ajouter un agent soluble dans les lipides à une émulsion de lipides selon l'une quelconque des revendications 1 à 10.

13. Procédé de préparation d'une composition pour injection intraveineuse, qui comprend l'étape d'ajouter un agent osmotique à une émulsion de lipides selon la revendication 1, pour obtenir une osmolarité comprise entre 280 et 300 milliosmoles.

14. Procédé de préparation d'une composition pour injection intraveineuse, comprenant l'étape de mélanger une émulsion de lipides selon l'une quelconque des revendications 1 à 11, avec une solution d'acides aminés et une solution de dextrose.

15. Utilisation d'une émulsion de lipides comprenant un émulsifiant, une huile de glycéride et de l'eau, le rapport pondéral de l'émulsifiant à l'huile de glycéride étant inférieur ou égal à 0,04, pour la préparation d'un médicament pour l'injection intraveineuse de lipides à un patient, tout en évitant l'hyperlipidémie.

16. Utilisation, selon la revendication 15, dans laquelle le patient est un nouveau-né ou un petit enfant.

FIG. 1

FIG. 2

EP 0 313 617 B1

FIG. 3